# EUROPEAN PATENT APPLICATION

(11) **EP 1 197 565 A1**
(43) Date of publication of application: **17.04.2002**
(21) Application number: 00122133.2
(22) Date of filing: 12.10.2000
(51) Int. Cl.: C12Q 1/68

(54) **A novel point mutation in the 3'region of the prothrombin gene associated with increased risk of thrombosis**

(71) Applicant: Roche Diagnostics GmbH, 68305 Mannheim (DE)
(72) Inventor: Wielckens, Klaus Prof. Dr. med., 50668 Köln (DE)

(57) **Abstract**

The invention relates to methods for screening a patient for increased risk of thrombosis, wherein a previously undiscovered mutation at position 20221 of the Prothrombin gene is detected.

## Description

The invention relates to the field of determination of hereditary thrombophilia by means of detecting polymorphisms in the Prothrombin Gene.

### Prior Art

As a multifactorial disease, both genetic factors and acquired conditions can increase the risk of developing venous thrombosis. While conditions such as pregnancy or immobilization have been known for decades to increase the likelihood for the development of venous thrombosis, only a few genetic factors have been identified recently. To date, two frequent genetic variations have been identified as important risk factors. In 1994 a mutation in the factor V gene (Leiden mutation) was shown to increase the risk 5- to 10-fold (1, 2, reviewed in 3) for developing venous thrombosis in heterozygotes. In 1996 a mutation at position 20210, located in the 3' untranslated region of the prothrombin gene, was shown to increase the risk for developing venous thrombosis almost threefold in heterozygotes(4).
The underlying mechanism of this prothrombin mutation is still not known, but heterozygote carriers of this mutation display on average a 20% increased prothrombin activity as compared to wild-type individuals, with prothrombin activity of heterozygotes and normal individuals greatly overlapping (4). Whether increased mRNA production, splicing efficiency or mRNA- and/or protein stability is responsible for these elevated prothrombin levels, has not yet been proven.
The prothrombin mutation 20210 is an independent risk factor, not only in adults but for childhood thrombophilia as well, though nongenetic triggers are discussed to play an important role as well (5,6,7).
Recently a case report featuring two patients suggested that prothrombin mutation 20210 is a risk factor for early renal allograft thrombosis (8). A total of three transplants had to be removed due to massive thrombosis within less than 14 days after transplantation from an 18 and an 8-year-old female.

Several techniques, including allele-specific PCR (9,10), heteroduplex analysis (11) and single-strand conformational polymorphism (12), have been described over the last years for the detection of point mutations. However, to date, RFLP (restriction fragment length polymorphism) still remains the most widely used technique (4). While this simple technique is easy to perform and requires only small upfront investments into hardware, it recognizes only those mutations which occur within the few basepairs of a restriction site. Therefore, mutations other than the ones expected and thus previously unknown, remain undetected even if they are in close proximity to the known mutation itself.

In addition to the mutation at position 20210 of the Prothrombin gene, a further mutation at position 20122 has been discussed as being putatively associated with an increased risk for thrombisis (13).

Summarizing, according to prior art a mutation at position 20210 in the Prothrombin gene so far was the only polymorphism which had been identified to be clearly associated with hereditary thrombophilia in this gene. Therefore, the object of the present invention was to identify further polymorphisms associated with increased risk of thrombosis and provide methods for detecting them.

### Brief description of the invention

Accordingly, the present invention is directed to the detection of a so-far unknown mutation in the prothrombin gene at nucleotide position No. 20221, which is in close proximity to the known mutation 20210. The new mutation seems to mimic the effects of the known prothrombin mutation 20210. This position no. 20221 corresponds to position no. 26795 as compared to the prothrombin sequence found in the EMBL data base (Accession no. HSTHBM 17262.1).
Therefore, The invention provides a method for screening a patient for increased risk of thrombosis, wherein a mutation at position 20221 in the Prothrombin gene is detected. In a preferred embodiment, the mutation is detected using at least one oligonucleotide hybridization probe. In a still even more preffered embodiment, 2 adjucently hybridizing probes are used, where each probe is labeled with a fluourphore and hybridization is detected by means of fluorescence resonance energy transfer (FRET). In has been proven to be advantageous, if the mutation is detected by means of melting curve analysis.

In alternative embodiments, the mutation is detected by means of sequencing, allele specific amplification , RFLP or SSCP.

### Brief description of drawing

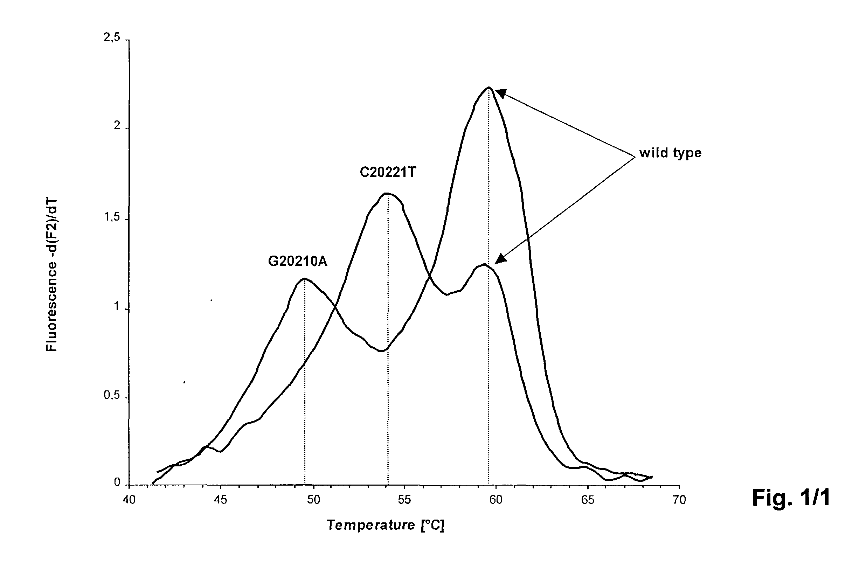

Fig.1: Melting curve analysis of the prothrombin 3'untranslated region of amplified genomic DNA obtained from heterozygous patients with mutations in nucleotide positions no 20210 and 20221.

### Detailed description of the invention

A 9-year old boy (O.E.) of lebanese/syrian origin, who suffered from an infectious disease at the age of 11 months and subsequently developed chronic renal failure resulting in regular dialysis, received an allogeneic renal transplant.

Seven days after receiving the transplant, O.E. developed an acute vascular rejection of his renal transplant. Doppler ultrasonography showed absence of venous flow in certain areas, indicative of intrarenal segmental vein thrombosis. As part of a routine procewdure the patient underwent immediate plasmapheresis. During this plasmapheresis he suffered grand mal seizure. A subsequent Magnetic Resonance Spectroscopy (MR) of the brain displayed multiple signs of thromboembolic infarction, whic initiated an extensive search for different thromboembolic risk factors.

With the results for antithrombin (AT III), protein C and S activity were within the normal range and the absence of the Leiden mutation, some of the most frequent risk factors for thrombophilia could be excluded.

When the patient's DNA was tested for the presence of the prothrombin mutation 20210 on the Light Cycler (Roche molecular Biochemicals) using the melting point temperature analysis software. For such an assay, the target nucleic acid was amplified in a typical PCR reaction with suitable amplification primers using the manufaturer's Prothrombin kit (see examples). The hybridization probes of the kit were present during the amplification reaction. They were designed as a pair of fluorescently labeled oligonucleotides which together are capable of acting according to the FRET-Hybprobe formate (WO 97/46707). After completion of the PCR-reaction, the temperature of the sample was constitutively increased, and fluorescence was detected as long as the hybridization probe was bound to the target DNA. At melting temperature, the hybridization probe was released from its target, and the fluorescent signal was decreasing immediately down to the background level. This decrease was monitored with an appropiate temperature-time plot, such that an exact temperature value could be determined, at which the temperature decrease was observed.

For the investigated patient, an atypical melting curve was obtained (Fig. 1). This melting curve displayed two melting points. While the melting point at 60 C° is typical for the wild type allele, the second melting point at 54 C° neither represents the wild type nor the prothrombin mutation 20210 which usually gives a melting point at 49 C°. The test was repeated with independently drawn samples and produced the same melting curve.
Sequencing of a 427 bp long fragment of both strands of the patient's DNA demonstrated a C → T exchange 11 bp downstream of the previously observed mutation 20210 at position 20221 on one allele while the other allele was wild type. This new mutation wasd termed "Cologne-Mutation".

Taking into account the elevated prothrombin levels in patients carrying the mutation at nucleotide 20210, determination of the prothrombin activity of the patients's plasma revealed a level of 119 % as compared to the average level of healthy individuals.

The 40 year old father and one 5-year-old sister were tested to be heterozygous for the mutation. The result was obtained by sequencing both strands of both DNA samples. Prothrombin levels were 161 % for both, the father and the daughter. The mother and two sisters displayed a wild type genotype.

Up to date, the mechanism is not known through which the prothrombin mutations 20210 and 20221 exert its effects that finally leads to an elevated prothrombin level. It has been speculated by Poort et al. (4) that mutation 20210 leads to an increased translation efficiency or increased mRNAstability. This assumption is based on the fact that the mutation is located only 14 bases downstream of the poly-A signal. It is known that changes in the poly-A signal itself, in the nucleotide to which poly-A is added or in the sequence adjacent to the poly-A signal can affect translation efficiency or mRNA stability (14,15). Since cleavage and subsequent polyadenylation usually occur 15 to 30 bases downstream of the poly-A signal (16) the same mechanism/effects might be responsible in case of the Cologne mutation which is located 25 bases downstream of the poly-A signal.

Besides Melting curve analysis and sequencing, there exist several other possibilities which according to the present invention are suitable in order to detect the new mutation indicative for hereditary thrombosis.

Usually in a first step DNA from the patient to be investigated needs to be isolated by conventional sample preparation techniques well known to people skilled in the art. The DNA can be either genomic DNA or cDNA derived from a sample of cellular RNA. In a second step, DNA from the Prothrombin gene locus needs to be amplified by conventional nucleic acid amplification technologies, for example by the well known Polymerase Chain Reaction (PCR).

In order to detect a mutated allele, the PCR product may simply be hybridized with an allele specific oligonucleotide hybridization probe which discriminates between wild type and mutant allele.

Alternatively, the amplified DNA may subsequently be digested with the Restriction Enzyme MwoI (also termed Bspl4). Subsequent Gel electrophoresis enables detection of patients carrying the 20221 mutation due to different band patterns obtainable from wild type and mutant alelles (4).

In another embodiment, the PCR product is analyzed be SSCP analysis (12). Briefly, the double stranded PCR product is thermally denatured into single strands, subsequently cooled down very rapidly in order to generate intramolecular secondary structures and subjected to native gel electrophoresis, wherein the seconday structures of wild type and mutatn sequences can be discriminated.

In yet another embodiment, the amplification of the Prothrombin sequence may be performed as allele specific amplification (EP 0 332 435). In order to achieve this, one of the amplification primers only hybridizes to the mutant allele but not to the wild type sequence. Prefereably, the 3' terminal nucleotide of the primer is complementary to the position of the mutated base. As a consequence, amplification only occurs with the sequence containing the mutation but not with the wild type sequence.

The following examples disclose the invention further:

### Example 1: Coagulation test

Blood was collected from the anticubital vein into Monovette tubes (Sarstedt, Nümbrecht, Germany) containing 0,106 mmol/L trisodium citrate. Plasma was prepared by centrifugation for 10 minutes at 3,000 g at room temperature, stored at -70°C and thawed immediately before testing. Antithrombin activity was measured with "Berichrom® Antithrombin III (A)" (Dade Behring, Marburg, Germany), protein C activity with "Protein C Reagent, coagulometric" (Dade Behring, Marburg, Germany) and protein S activity with "Protein S Clottin Test" (Roche, Mannheim, Germany). Prothrombin activity was determined using a one-step clotting assay with Innovin® (Dade Behring, Marburg, Germany) and Factor II deficient plasma (Dade Behring, Marburg, Germany). All assays were performed on the automated Behring coagulation system (BCS) (Dade Behring, Marburg, Germany).

### Example 2: Sequence analysis

Genomic DNA was extracted from EDTA-blood using the High-Pure PCR-Template Preparation Kit (Roche Diagnostics, Mannheim, Germany). The sequence of the prothrombin gene was amplified by PCR, using sequence-specific primers (MWG-Biotech AG, Ebersberg, Germany) with the following sequences: According to nucleotide positions no. 26438-264555' GG AGA AAC-3' (forward) and 26843-26865 A-3' (reverse). **(EMBL Gene Bank Acc.No. HSTHBM17262.1)** Each reaction mixture of 50 µl contained: 50 ng genomic DNA, 1.5 mM MgCl₂ (Sigma, USA), 10x PCR-Reaction Buffer (Sigma, USA), 200 µM dNTPs (Promega, USA), 10 pmol primer and 0.5 U Taq polymerase (Sigma, USA). 33 cycles were performed on a thermocycler (Tri-Block-Thermocycler, Biometra) with the following cycle conditions: initially 2 min at 96°C, then 33 cycles: 1 min at 58°C, 1 min at 72°C and 1 min at 96°C. Yield and size of the amplicons were evaluated by agarose gel electrophoresis.

PCR products were then subjected to sequencing analysis. Prior to sequencing the probes were purified using High-Pure PCR-Product Preparation Kit (Roche, Mannheim, Germany). For cycle sequencing, PCR primers were used to generate the 5'moiety and 3'moiety using AmpliTaq-FS Big Dye Terminators (Perkin Elmer Applied Biosystems, USA) with the following cycle conditions: 10 sec at 96°C, 5 sec at 58°C, 4 min at 60°C, 25 cycles. Sequencing products were then resolved on an ABI 377 (Perkin Elmer Applied Biosystems, USA) automated sequencer with a Perkin Elmer sequencing analysis software version 2.1.1. All genetic variants were sequenced in both directions and confirmed by further independent genomic amplifications and sequencing reactions.

### Example 3: Mutation Detection with melting curve analysis

Genomic DNA from probands was extracted from EDTA-blood using the High-Pure PCR-Template Preparation Kit (Roche Diagnostics, Mannheim, Germany). The PCR was performed on a LightCycler Real Time PCR instrument (Roche Diagnostics, Mannheim, Germany) using the commercial LightCycler-Prothrombin Mutation Detection Kit (Roche Diagnostics, Mannheim, Germany). Each reaction mixture of 20 µl volume contained: 40 ng genomic DNA, 2 µl of 10x LightCycler-Prothrombin Reaction Mix and 2 µl of 10x LightCycler-Prothrombin Mutation Detection Mix. 45 cycles were performed according to the manufacturer's protocol on the LightCycler with cycle conditions as listed: initially 30 sec at 95°C, then 45 cycles with 10 sec at 55°C, 5 sec at 72°C and 1 sec at 95°C. The melting curve analysis proceeded for 60 sec at 95°C, 30 sec at 55°C and 30 sec at 45°C, finally 120 sec at 40°C and increased in 0,1°C/sec steps to 70°C final temperature.

### References

1. Bertina RM, Koeleman BP, Koster T, Rosendaal FR, Dirven RJ, de Ronde H, van der Velden PA, Reitsma PH. Mutation in blood coagulation factor V associated with resistance to activated protein C. Nature 1994; 369: 64-7.
2. Rees DC, Cox M, Clegg JB. World distribution of factor V Leiden. Lancet 1995; 346:1133-4.
3. Zöller B. Familial Thrombophilia-Resistance to activated protein S deficiency. [Thesis] Malmö, Sweden: University of Lund, Department of Clinical Chemistry, 1996.
4. Poort SR, Rosendaal FR, Reitsma PH, Bertina RM. A common genetic variation in the 3'-untranslated region of the prothrombin gene is associated with elevated plasma prothrombin levels and an increase in venous thrombosis. Blood 1996; 88:3698-703.
5. Nowak-Gottl U; Koch HG; Aschka I; Kohlhase B; Vielhaber H; Kurlemann G; Oleszcuk-Raschke K; Kehl HG; Jurgens H; Schneppenheim R. Resistance to activated protein C (APCR) in children with venous or arterial thromboembolism . Br J Haematol 1996; 92: 992-8
6. Nowak-Gottl U; Kohlhase B; Vielhaber H; Aschka I; Schneppenheim R; Jurgens H. APC resistance in neonates and infants: adjustment of the APTT-based method. Thromb Res 1996; 81: 665-70
7. Nowak-Gottl U; Dubbers A; Kececioglu D; Koch HG; Kotthoff S; Runde J; Vielhaber H. Factor V Leiden, protein C, and lipoprotein (a) in catheter-related thrombosis in childhood: a prospective study. J Pediatr 1997; 131: 608-12
8. Oh J; Schaefer F; Veldmann A; Nowak G; Nowak-Gottl U; Tonshoff B; Kreuz W. Heterozygous prothrombin gene mutation: a new risk factor for early renal allograft thrombosis. Transplantation 1999; 68: 575-8
9. Hellgren M, Svensson PJ, Dahlbäck B. Resistance to activated protein C as a basis for venous thromboembolism associated with pregnancy and oral contraceptives. Am J Obstet Gynecol 1995; 173:210-3.
10. Hezard N, Cornillet-Lefebvre P, Gillot L, Potron G, Nguyen P. Multiplex ASA PCR for a simultaneous determination of factor V Leiden gene, G→A 20210 prothrombin gene and C→T 677 MTHFR gene mutations. [letter] Thromb Haemost 1998; 79: 1054-5.
11. Bowen DJ, Bowley S, John M, Collins PW. Factor V Leiden (G1691A), the prothrombin 3'-untranslated region variant (G20210A) and thermolabile methylenetetrahydrofolate reductase (C677T): a single genetic test genotypes all three loci--determination of frequencies in the S. Wales population of the UK. Thromb Haemost 1998; 79: 949-54.
12. Aschka I, Bergmann F, Budde U, Eberl W, Eckhof-Donovan S, Kreuz W, Nowak-Göttl U, Plendl H, Schobeß R, Schneppenheim R. Prevalence of APC-resistance in children. [abstract]. Thromb Haemost 1995; 73:1121.
13. Meyer M, Kutscher G and Vogel G. The Prothrombin nt20210 A Allele as a Risk Factor for Venous Thromboembolism: Detection of Heterozygous and Homozygous Carriers by Alternative Methods. Thromb Res 2000; 97: 359-63.
14. Sheets MD, Ogg SC, Wickens MP. Point mutations in AAUAAA and the poly (A) addition site: Effects on the accuracy and efficiency of cleavage and polyadenylation in vitro. Nucleic Acids Res. 1990; 18; 5799-805
15. McLauchlan J, Gaffney D, Whitton JL, Clements J.B. The consensus sequence YGTGTTYY located downstream from the AATAAA signal is required for efficient formation of mRNA 3'termini. Nucleic Acids Res. 1985; 13: 1347-68.
16. Proudfoot NJ, Brownlee GG. 3'non-coding region sequences in eukaryotic messenger RNA. Nature 1976; 263:211-4.

## Claims

1. Method for screening a patient for increased risk of thrombosis, wherein a mutation at position 20221 in the Prothrombin gene is detected.

2. Method according to claim 1 wherein the mutation is detecting using at least one oligonucleotide hybridization probe

3. Method according to claim 1 or 2 wherein the mutation is detected by means of melting curve analysis

4. Method according to claim 1, wherein the mutation is detected by means of allele specific amplification

5. Method according to claim 1, wherein the mutation is detected by means of SSCP

6. Method according to claim 1, wherein the mutation is detected by means of RFLP

7. Method according to claim 1, wherein the mutation is detected by means of sequencing
